# FASCICULE DE BREVET EUROPEEN

(11) **EP 0 479 688 B1**
(45) Date de publication et mention de la délivrance du brevet: **01.03.1995**
(21) Numéro de dépôt: 91420341.9
(22) Date de dépôt: 27.09.1991
(51) Int. Cl.: A61B 5/22, A63B 21/00

(54) **Appareil de mesure d'un effort musculaire**
Einrichtung zur Erfassung der Muskelkraft
Apparatus for measuring muscular force

(30) Priorité: 01.10.1990 FR 9012375
(43) Date de publication de la demande: 08.04.1992
(73) Titulaire: CENTRE STEPHANOIS DE RECHERCHES MECANIQUES HYDROMECANIQUE ET FROTTEMENT Société anonyme, F-42166 Andrezieux Boutheon Cédex (FR)
(72) Inventeur: Berger, Antoine, F-42000 Saint Etienne (FR); Barlerin, Jean-Claude, F-42170 Saint.Just.Rambert (FR)
(74) Mandataire: Dupuis, François

(56) Documents cités:
- EP-A- 0 151 066
- EP-A- 0 267 071
- WO-A-88/01185
- PATENT ABSTRACTS OF JAPAN vol. 9, no. 79 (P-347)(1802) 9Avril 1985 & JP-A-59 211 163 ( TOYOTA JIDOSHA K. K. ) 29 Novembre 1984

## Description

L'invention concerne plus particulièrement un appareil pour la rééducation et/ou le développement d'un muscle notamment, appartenant à un membre supérieur et/ou inférieur du corps humain.

On connaît de nombreux appareils conformés pour remplir de telles fonctions. Généralement, la plupart de ces appareils mettent en oeuvre des moyens du type ressort ou autres susceptibles de créer une force antagoniste réglable ou non qu'il convient de vaincre. L'intensité de la force exercée peut être affichée sur des écrans. Par ailleurs ces appareils, en fonction notamment du but recherché, peuvent fonctionner soit en statique, soit en dynamique.

On connaît des appareils comprenant un organe sollicité par une partie du corps humain pour subir une force de traction et/ou de poussée, cet organe étant asservi par des moyens indiquant l'intensité de la force exercée. Cet état de la technique peut être illustré par l'enseignement des Brevets WO-A-8801185, EP-A-0.151.066 et EP-A-0.267.271.

Quelle que soit la conception de ce type d'appareils, après relâchement de l'effort exercé, il se produit une force de réaction qui est plus ou moins transmise au muscle concerné. Or dans certains cas, notamment pour des entraînements musculaires spécifiques, il s'avère important de ne pas soumettre le muscle à une quelconque force, après relâchement de l'effort exercé. C'est le cas notamment en escalade, au niveau des muscles des mains.

Bien évidemment, ce problème se pose plus particulièrement dans le cas d'une mesure d'effort en dynamique.

Pour remédier aux inconvénients des appareils de musculation et/ou de rééducation connus, le problème que se propose de résoudre l'invention, est de mesurer un effort sans pour autant soumettre le muscle considéré à une quelconque force de réaction.

Le problème posé de mesurer l'effort musculaire sans pour autant soumettre le muscle à une force de réaction lorsque l'effort est relâché dans le cas d'un fonctionnement dynamique, est résolu en ce que l'organe est monté avec capacité de déplacement en étant assujetti à un dispositif apte à soumettre ledit organe à un mouvement continu de va et vient de vitesse et de course réglables, seulement lorsqu'une valeur de seuil déterminé et réglable correspondant à l'intensité d'une force est atteinte, ledit organe demeurant immobile, ou s'arrêtant lorsque ladite valeur n'est pas atteinte.

Le problème posé de déplacer l'organe mobile seulement lorsque la force exercée sur ce dernier est au moins égale à la valeur d'un seuil déterminé, ce qui a pour effet, inversement, de provoquer l'arrêt dudit mobile aussitôt que l'effort est relâché, est résolu en ce que le dispositif comprend un ensemble de mesure constitué d'un moteur monté avec capacité de déplacement limité en étant accouplé à un organe capteur fixe asservi à une centrale de commande, ledit moteur présentant des agencements pour assurer ou non le déplacement du ou des organes soumis à la force de traction et/ou de poussée, .

Avantageusement, ce problème posé est résolu en ce que les agencements du moteur sont constitués par une vis sans fin engagée dans une partie d'un support faisant office d'écrou et équipé du ou des organes.

Le support est monté avec capacité de déplacement en translation guidé sur une embase.

En fonction du muscle à rééduquer ou à développer, le support équipé du ou des organes est monté avec capacité de déplacement par rapport à une semelle d'appui fixe agencée pour recevoir la partie du corps humain concernée.

Compte tenu du problème posé à résoudre, la centrale de commande comprend, en combinaison, différents modules aptes à assurer les fonctions essentielles suivantes :
- affichage d'un seuil d'effort correspondant à une force d'intensité déterminée et variable ;
- démarrage du moteur si la valeur de la force exercée sur l'organe est au moins égale ou supérieure à la valeur du seuil affiché provoquant le déplacement suivant un mouvement de va et vient continu du support équipé du ou des organes ;
- mesure de l'effort fourni ;
- mesure de la vitesse de déplacement et de la course ;
- mesure de la durée de l'effort.

La figure 1 est une vue en coupe à caractère schématique montrant un exemple de réalisation de l'appareil de mesure mettant en oeuvre le principe à la base de l'invention. Dans cette figure, l'organe sollicité par une partie du corps humain est à l'état repos.

La figure 2 est une vue semblable à la figure 1 montrant le déplacement de l'organe mobile lorsqu'un effort est exercé sur ce dernier et lorsque l'intensité de cet effort est supérieur à la valeur d'un seuil prédéterminé.

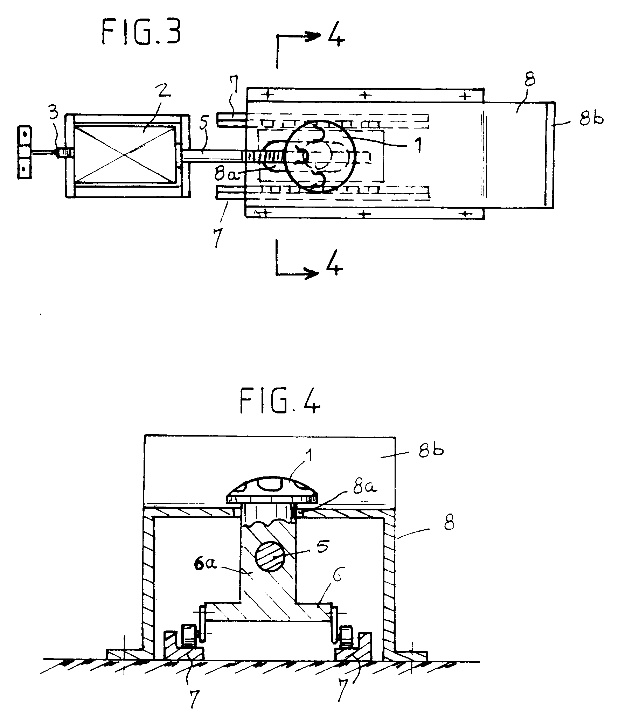

La figure 3 est une vue en plan correspondant à la figure 1.

La figure 4 est une vue en coupe considérée selon la ligne 4-4 de la figure 3.

L'appareil de mesure selon l'invention comprend au moins un organe (1) conformé pour être sollicité par une partie du corps humain pour subir une force de traction et/ou de poussée. Comme il sera indiqué dans la suite de la description, cet organe (1) peut présenter différentes formes de réalisation en fonction des applications envisagées et de la partie du corps humain concernée et qui peut être constituée par une partie de l'un des membres supérieurs ou inférieurs par exemple.

Cet organe (1), quelle que soit sa forme de réalisation, est asservi par des moyens aptes à indiquer l'intensité de la force exercée.

Dans une forme de réalisation préférée, l'organe (1) est monté avec capacité de déplacement, notamment en translation, en étant assujetti à un dispositif apte à provoquer son déplacement selon un mouvement de va et vient continu en créant une force de réaction (F) opposée à la force (F1) exercée sur ledit organe. Le dispositif est par ailleurs conformé pour assurer le déplacement de l'organe (1) selon une vitesse et une course réglables.

Plus particulièrement, selon une caractéristique à la base de l'invention, le déplacement de l'organe (1) est autorisé seulement lorsque la force (F1) qu'il subit est supérieure ou au moins égale à une valeur de seuil déterminée et réglable. Lorsque cette valeur de seuil n'est pas atteinte, l'organe (1) demeure immobile ou bien s'arrête immédiatement lorsque l'intensité de la force (F1) exercée repasse en-dessous de cette valeur de seuil.

Pour l'essentiel, et dans la forme de réalisation illustrée, le dispositif, auquel est asservi l'organe (1) et permettant son déplacement seulement dans les conditions précitées, comprend un ensemble de mesures constitué par un moteur (2) monté avec capacité de déplacement limité en étant accouplé à un organe capteur fixe (3). Cet organe capteur (3), de tout type connu et approprié et dont la conception dépend de la nature de la force exercée, à savoir soit une force de poussée, soit une force de traction, est asservi à une centrale électronique de commande (4).

Le moteur (2) est en outre agencé pour assurer ou non le déplacement du ou des organes soumis à la force de traction et/ou de poussée, selon un mouvement de va et vient continu. Par exemple, l'arbre du moteur entraîne une vis sans fin (5) engagée dans une partie (6a) d'un support (6) recevant l'organe (1). Cette partie (6a) fait office d'écrou et est immobilisée en rotation, de sorte que l'entraînement de la vis sans fin (5) par le moteur (2), assure le déplacement concomitant de l'ensemble du support. Le support (6) est monté avec capacité de déplacement en translation guidée sur une embase (7).

Ce mode de déplacement par un système vis-écrou tel que défini, assure une grande réduction et permet l'irréversibilité du mouvement. Cependant, ce système ne doit pas être considéré comme limitatif. En effet, tout autre système provoquant un déplacement linéaire, tel que pignon-crémailière, vérin hydraulique ou autres peut entrer dans le cadre de l'invention.

Comme indiqué, le dispositif de déplacement provoque un aller-retour de l'organe mobile (1), la course et la vitesse de ce mouvement étant réglables par l'opérateur.

Avantageusement, le support (6) de l'organe (1) est monté avec capacité de déplacement par rapport à une semelle d'appui fixe (8) agencée pour recevoir la partie du corps humain concernée. Par exemple, le support (1) se déplace dans une lumière (8a) de la semelle d'appui (8). Comme indiqué, cette semelle d'appui présente tous types d'agencements permettant de faire reposer la partie du corps humain concernée et destinée à solliciter, en poussée ou en traction, l'organe (1).

La centrale de commande (4) comprend, en combinaison, différents modules aptes à assurer les fonctions essentielles suivantes :
- affichage d'un seuil d'effort correspondant à une force d'intensité déterminée et variable ;
- démarrage du moteur (2) si l'intensité de la force (F1) exercée sur l'organe (1) est au moins égale ou supérieure à la valeur du seuil affiché, provoquant ainsi, sous l'action de la vis sans fin (5), le déplacement du support (6) selon un mouvement continu de va et vient ;
- mesure de l'effort fourni ;
- mesure de la vitesse de déplacement et de la course de l'organe (1) ;
- mesure de la durée de l'effort (F1) appliqué.

L'appareil de mesure tel que décrit trouve de nombreuses applications pour la rééducation ou le développement d'un muscle.

Notamment, l'appareil trouve une application particulièrement avantageuse pour l'entraînement à l'escalade,.au niveau des muscles des mains qui sont très sollicités dans ce type de sport. Dans ce cas, l'organe (1) est constitué par une forme présentant différentes empreintes pour constituer une prise d'escalade artificielle. La semelle d'appui (8) présente une partie en relief (8b) faisant office de butée et permettant le calage de l'avant-bras et/ou du corps au niveau de l'aisselle et de l'épaule, lorsque la main saisit la prise d'escalade (1).

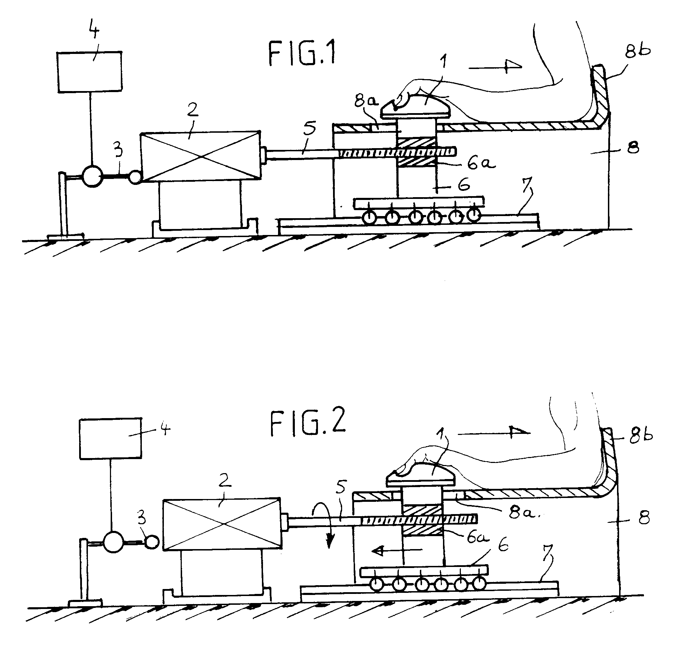

Il en résulte le cycle suivant:
L'utilisateur affiche la valeur du seuil correspondant à l'intensité de la force de traction qu'il souhaite dépasser. Après avoir positionné l'avant-bras sur la partie correspondante de la semelle (8), et disposé les doigts de la main dans les empreintes de la prise d'escalade (1), l'utilisateur exerce sur ladite prise un effort de traction (F1) ce qui a pour effet de solliciter le capteur de mesure (3). Si, en fonction de l'état du capteur (3), cet effort (F1) est inférieur à la valeur du seuil affiché, le moteur (2) n'est pas entraîné et par conséquent l'ensemble du support (6) recevant la prise (1) est immobile (figure 1). Si cet effort (F1) atteint et dépasse la valeur du seuil affichée, le moteur (2) est entraîné provoquant de manière concomitante, sous l'effet de la vis sans fin (5), le déplacement du support (6) équipé de la prise (1) en créant une force de réaction (F) opposée à la force de traction exercée (F1). Le déplacement du support (6), et par conséquent de la prise (1), s'effectue selon un mouvement continu de va et vient dont la course et la vitesse peuvent être réglables par l'utilisateur.

A noter, lorsque l'effort va dans le sens du déplacement du support, les muscles travaillent en concentrique. Inversement, lorque l'effort est opposé au sens de déplacement, les muscles travaillent en excentrique.

Dès que l'utilisateur relâche son effort, ou que l'intensité de la force (F1) redevient inférieure à la valeur du seuil affichée, le moteur (2) n'est plus entraîné, et par conséquent le support (6) recevant la prise (1) est immédiatement arrêté.

Les avantages ressortent bien de la description, en particulier on souligne et on rappelle la possibilité d'exercer un effort musculaire et de ne plus soumettre le muscle concerné à un éventuel effet de réaction, après relâchement dudit effort exercé.

## Revendications

1. Appareil de mesure d'un effort musculaire notamment, comprenant au moins un organe (1) conformé pour être sollicité par une partie du corps humain pour subir une force de traction et/ou de poussée, caractérisé en ce que l'organe (1) est monté avec capacité de déplacement en étant assujetti à un dispositif apte à soumettre ledit organe (1) à un mouvement continu de va et vient, de vitesse et de course réglables, seulement lorsqu'une valeur de seuil déterminé et réglable correspondant à l'intensité d'une force est atteinte, ledit organe (1) demeurant immobile ou s'arrêtant alors que ladite valeur n'est pas atteinte.

2. Appareil selon la revendication 1, caractérisé en ce que le dispositif comprend un ensemble de mesure constitué d'un moteur (2) monté avec capacité de déplacement limité en étant accouplé à un organe capteur fixe (3) asservi à une centrale de commande (4), ledit moteur présentant des agencements pour assurer ou non le déplacement du ou des organes soumis à la force de traction et/ou de poussée.

3. Appareil selon la revendication 2, caractérisé en ce que les agencements du moteur sont constitués par une vis sans fin (5) engagée dans un partie (6a) d'un support (6) faisant office d'écrou et équipé du ou des organes.

4. Appareil selon la revendication 3, caractérisé en ce que le support (6) est monté avec capacité de déplacement en translation guidé sur une embase (7).

5. Appareil selon l'une quelconque des revendications 1 à 4, caractérisé en ce que le support (6) équipé du ou des organes (1) est monté avec capacité de déplacement par rapport à une semelle d'appui (8) fixe agencée pour recevoir la partie du corps humain concernée.

6. Appareil selon l'une quelconque des revendications 1 à 5, caractérisé en ce que la centrale de commande comprend en combinaison différents modules aptes à assurer les fonctions essentielles suivantes :
- affichage d'un seuil d'effort correspondant à une force d'intensité déterminée et variable ;
- démarrage du moteur (2) si la valeur de la force exercée (F1) sur l'organe (1) est au moins égale ou suprieure à la valeur du seuil affiché provoquant le déplacement selon un mouvement de va et vient continu du support (6) équipé du ou des organes (1) ;
- mesure de l'effort fourni ;
- mesure de la vitesse de déplacement et de la course ;
- mesure de la durée de l'effort.

7. Appareil selon l'une quelconque des revendications 1 à 6, et son application à la rééducation d'un muscle.

8. Appareil selon l'une quelconque des revendications 1 à 6, et son application au développement d'un muscle.

## Claims

1. Apparatus for measuring a muscular effort, in particular, comprising at least one component (1) shaped to be stressed by a part of the human body so that it is subjected to a tensile and/or thrust force characterised in that the component (1) is mounted so that it can move and is controlled by a device capable of subjecting said component (1) to a continuous to and fro movement of adjustable speed and travel only when an adjustable, predetermined threshold value equivalent to the strength of a force is reached, said component (1) remaining stationary or stopping if said value is not reached.

2. Apparatus as claimed in claim 1, characterised in that the device comprises a measuring assembly consisting of a motor (2) mounted with a limited displacement capacity and linked to a fixed sensor (3) controlled by a central control unit (4), said motor having features to ensure movement or no movement of the component(s) subjected to the tensile and/or thrust force.

3. Apparatus as claimed in claim 2, characterized in that the features of the motor consist of an endless screw (5) fitted in a part (6a) of a support (6) acting as a nut and equipped with the component(s).

4. Apparatus as claimed in claim 3, characterized in that support (6) is mounted with the capacity for guided translational movement on a base (7).

5. Apparatus as claimed in any of claims 1 to 4, characterized in that support (6) equipped with component(s) (1) is mounted with a capacity for movement with respect to a fixed rest (8) devised to accommodate that part of the human body in question.

6. Apparatus as claimed in any of claims 1 to 5, characterized in that the central control unit comprises a combination of various modules capable of fulfilling the following essential functions:
- display of an effort threshold equivalent to a force of predetermined, variable strength;
- starting of motor (2) if the value of the force exerted (F1) on component (1) is at least equal to or greater than the value of the displayed threshold causing a continuous to and fro movement of support (6) equipped with component(s) (1):
- measurement of effort exerted;
- measurement of speed of movement and travel;
- measurement of duration of effort.

7. Apparatus as claimed in any of claims 1 to 6 and its application to the rehabilitation of a muscle.

8. Apparatus as claimed in any of claims 1 to 6 and its application to the development of a muscle.

## Patentansprüche

1. Gerät zur Messung insbesondere der Muskelkraft mit mindestens einem Bauteil (1), das für die Zug- und/oder Druckbeanspruchung durch einen Teil des menschlichen Körpers ausgebildet ist, dadurch gekennzeichnet, daß das Bauteil (1) verschiebbar gelagert ist, wobei es von einer Vorrichtung abhängt, die geeignet ist, auf das Bauteil (1) eine kontinuierliche Hin- und Herbewegung mit verstellbarer Geschwindigkeit und verstellbarem Laufweg auszuüben, und zwar nur dann, wenn ein festgelegter und regulierbarer, einer bestimmten Kraftintensität entsprechender Schwellenwert erreicht ist, wogegen das Bauteil (1) bewegungslos verharrt oder stehenbleibt, wenn der besagte Wert nicht erreicht wird.

2. Gerät nach Anspruch 1, dadurch gekennzeichnet, daß die Vorrichtung eine Meßeinrichtung bestehend aus einem Motor (2) umfaßt, der in begrenztem Umfang verschiebbar und dabei mit einem ortsfesten Meßfühler (3) gekoppelt ist, welcher von einer Schaltzentrale (4) gesteuert wird, wobei der Motor Vorkehrungen für die Verschiebung bzw. Nichtverschiebung des/der von der Zug- und/oder Druckkraft beaufschlagten Bauteils/-e aufweist.

3. Gerät nach Anspruch 2, dadurch gekennzeichnet, daß die Vorkehrungen des Motors aus einer Schnecke (5) bestehen, die in einen Teil (6a) eines Trägers (6) eingeführt ist, der als Mutter dient und mit dem/den Bauteil/-en bestückt ist.

4. Gerät nach Anspruch 3, dadurch gekennzeichnet, daß der Träger (6) verschiebbar auf einem Sockelelement (7) geführt wird.

5. Gerät nach einem der Ansprüche 1 - 4, dadurch gekennzeichnet, daß der mit dem/den Bauteil/-en (1) bestückte Träger (6) gegenüber einer ortsfesten Auflageplatte (8) für die Aufnahme des betreffenden Körperteils verschiebbar gelagert ist.

6. Gerät nach einem der Ansprüche 1 - 5, dadurch gekennzeichnet, daß die Schaltzentrale eine Kombination verschiedener Module umfaßt, die folgende Hauptfunktionen ausüben können:
- Anzeige eines Kraft-Schwellenwerts, der einer festgelegten und veränderlichen Kraftintensität entspricht;
- Anlaufen des Motors (2), wenn die auf das Bauteil (1) ausgeübte Kraft mindestens gleich oder größer ist als der angezeigte Schwellenwert, wodurch eine kontinuierliche Hin- und Herbewegung des mit dem/den Bauteil/-en bestückten Trägers (6) ausgelöst wird;
- Messung der aufgewendeten Kraft;
- Messung der Bewegungsgeschwindigkeit und des Laufwegs;
- Messung der Kraftdauer.

7. Gerät nach einem der Ansprüche 1 - 6 und dessen Anwendung auf die Rehabilitation eines Muskels.

8. Gerät nach einem der Ansprüche 1 - 6 und dessen Anwendung auf die Kraftenwicklung eines Muskels.
